(19) Europäisches Patentamt
European Patent Office
Office européen des brevets

(11) Publication number: **0 519 449 A1**

# (12) EUROPEAN PATENT APPLICATION

(21) Application number: **92110267.9**

(22) Date of filing: **17.06.92**

(51) Int. Cl.5: **C07D 417/12**, A61K 31/425, C07D 277/48, A61K 31/435, A61K 31/505, A61K 31/53

(30) Priority: **21.06.91 IT MI911714**
**01.04.92 IT MI910786**

(43) Date of publication of application:
**23.12.92 Bulletin 92/52**

(84) Designated Contracting States:
**PT**

(71) Applicant: **BOEHRINGER MANNHEIM ITALIA S.P.A.**
**Via S. Uguzzone, 5**
**I-20126 Milano(IT)**

(72) Inventor: **De Cillis, Gianpiero**
**c/o Via S. Uguzzone, 5**
**I-20126 Milano(IT)**
Inventor: **Long, Giorgio**
**c/o Via S. Uguzzone, 5**
**I-20126 Milano(IT)**
Inventor: **D'Alo, Simonetta**
**c/o Via S. Uguzzone, 5**
**I-20126 Milano(IT)**
Inventor: **Rozzi, Antonella**
**c/o Via S. Uguzzone, 5**
**I-20126 Milano(IT)**
Inventor: **Gallico, Licia**
**c/o Via S. Uguzzone, 5**
**I-20126 Milano(IT)**

(74) Representative: **Weber, Manfred, Dr. et al**
**Boehringer Mannheim GmbH**
**Patentabteilung Sandhofer Strasse 116**
**W-6800 Mannheim 31(DE)**

## (54) 2-Amino-4-aryl thiazoles with antiasthmatic and antiinflammatory activities on the respiratory tract.

(57) 2-Amino-4-aryl-thiazole derivatives have interesting pharmacological properties, particularly anthiasthmatic and antiinflammatory activities on the respiratory tract and they can be used to prepare pharmaceutical compositions useful for the treatment of bronchial hyper-reactivity.

EP 0 519 449 A1

The present invention relates to 2-amino-4-arylthiazole derivatives, a process for the preparation thereof and pharmaceutical compositions containing them.

More precisely the invention relates to compounds of formula (I)

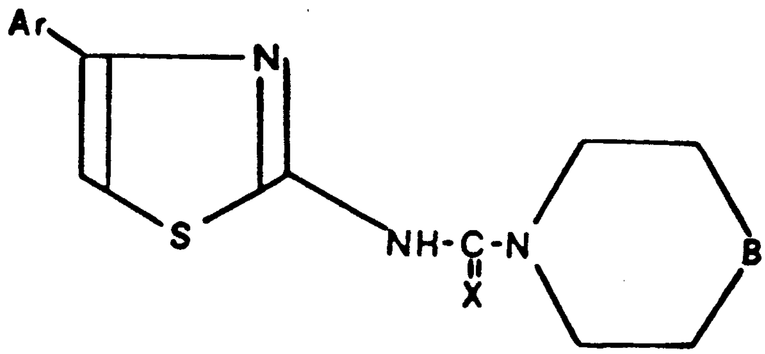

(I)

wherein:

**X** is oxygen or sulfur;

**B** is $CH_2$, oxygen, sulfur or N-R;

**R** is $C_1$-$C_6$ alkyl, phenyl optionally substituted with halogen in o-, m- or p-position; benzyl optionally substituted with halogen in the o-, m- or p- positions; bis(phenyl)methyl; bis(o-,m- or p-halophenyl)methyl; a 5-6 membered heterocycle having 1 to 3 nitrogen atoms, optionally substituted with 1-2 amino groups, mono-$C_1$-$C_6$-alkylamino, mono-$C_3$-$C_7$-alkenyl- or mono-$C_3$-$C_7$-alkynylamino, di-$C_1$-$C_6$-alkylamino, $C_1$-$C_6$-alkyl-$C_3$-$C_7$-alkenylamino, piperidin-1-yl, morpholin-4-yl, pyrrolidin-1-yl.

**Ar** is phenyl optionally substituted with 1-3 substituents selected from halogen atoms; hydroxy; $C_1$-$C_6$alkoxy; $C_1$-$C_6$ acyloxy; $C_1$-$C_6$ alkyl; cyano; nitro; $-SO_2CH_3$; $-SO_2NH_2$; phenylsulfonyl optionally substituted with halogen in the o-, m- or p-positions; $C_1$-$C_6$ alkylthio; phenyloxy or phenylthio optionally substituted with halogen in the o-, m- or p- positions; $CF_3$; NR'R", wherein R' and R", which may be the same or different, are hydrogen, $C_1$-$C_4$ alkyl, acetyl or NR'R" is a pyrrolidino, piperidino, morpholino, 4-thiamorpholino group; $-CH_2$NR'R" wherein R' and R" are as defined above; $-CO_2$R''' wherein R''' is hydrogen or $C_1$-$C_4$ alkyl; $-NH-SO_2$-R'''' wherein R'''' is methyl, ethyl, trifluoromethyl or Ar is $\alpha$, $\beta$ or $\gamma$-pyridyl or N-oxides thereof.

Ar is preferably phenyl or phenyl substituted with 1 or 2 hydroxy, $C_1$-$C_6$ alkoxy, amino, halogen, (halosubstituted) phenylsulphonyl, cyano, nitro, phenylthio, alkoxycarbonyl and/or $C_1$-$C_6$ alkyl groups, preferably tert-butyl groups, $-NHSO_2CH_3$.

Particularly preferred Ar groups are phenyl, 4-hydroxyphenyl, 3,4-dihydroxyphenyl, 2,3-dihydroxyphenyl, 4-hydroxy-3,5-di-tert-butylphenyl, 2-, 3- or 4-methoxyphenyl, 2-chlorophenyl, 3-chlorophenyl, 4-chlorophenyl, 2-, 3- or 4-fluorophenyl, 2-, 3- or 4-bromophenyl, 3- or 4-trifluoromethylphenyl, 3- or 4-cyanophenyl, 4-nitrophenyl, 3-nitrophenyl, 3- or 4-phenylsulphonyl-phenyl, 3- or 4-(2'-chlorophenyl)-sulphonylphenyl, 4-phenylthiophenyl, 4-((pyrrolidin-1-yl)methyl)phenyl, 2-, 3- or 4-aminophenyl, 3- or 4-methoxycarbonylphenyl, $\beta$-pyridyl, 3,4-dichlorophenyl, 4-methylsulfonylamino-3-phenoxy.

When R is an heterocycle, it is preferably selected from pyridin-2-yl, pyrimidin-4-yl, pyrimidin-2-yl or 1,3,5-triazin-2-yl, which can optionally be substituted with 1 or 2 amino, mono-$C_1$-$C_6$-alkylamino,2-propenylamino, 2-propynylamino, propylamino, isopropylamino, dimethylamino, diethylamino, ethyl-2-propenylamino, pyrrolidino groups.

Most preferably, R is selected from:

[2,6-bis(diethylamino)pyrimidin-4-yl],
[2,6-bis(2-propenylamino)pyrimidin-4-yl],
[2,6-bis(amino)pyrimidin-4-yl],
[2,6-bis(pyrrolidin-1-yl)pyrimidin-4-yl],
[4,6-bis(pyrrolidin-1-yl)pyrimidin-2-yl],
[4,6-bis(2-propenylamino)-1,3,5-triazin-2-yl],
[4,6-bis(diethylamino)-1,3,5-triazin-2-yl],
[4,6-bis(pyrrolidin-1-yl)-1,3,5-triazin-2-yl],
[3,6-bis(diethylamino)pyridin-2-yl],
[3,6-bis(pyrrolidin-1-yl)pyridin-2-yl],

[3,6-bis(2-propenylamino)pyridin-2-yl],
methyl, p-fluorophenyl, p-chlorophenyl, p-bromophenyl, phenyl, bis(phenyl)methyl, bis(p-fluorophenyl)-methyl, bis(p-chlorophenylmethyl), bis(p-bromophenylmethyl).

The present invention also relates to the salts of compounds of formula (I) with pharmaceutically acceptable acids.

The compounds of formula (I) have interesting pharmacological properties, particularly antiasthmatic and antiinflammatory activities on the respiratory tract. The compounds of the invention show moreover marked antihistaminic effect.

5-Phenyl-2-aminothiazole derivatives having antiinflammatory activity are disclosed in WO 8202383, 8202384, 8202385, 8202386 and 8202553.

EP-A-0032058 discloses 5-aminoalkyl-2-aminothiazoles having antiallergic and antiasthmatic activities, whereas 2-amino-4-methyl-5-(4-phenyl)piperazino-alkylthiazoles having neuroleptic activity are disclosed in Polish patent 106,675 (Chem. Abstr. 95; 97851).

Finally, 4-phenyl-2-aminothiazole derivatives have been studied as local anaesthetic in J. Indian Chem. Soc. 1980, 57, pp 829-832 and 1982, 59, pp 773-5.

The compounds of formula I structurally differ from the prior-art thiazoles in the kind of substitution on the amine nitrogen. From the biological point of view, the distinguishing feature of the compounds of the invention resides in their particular ability in preventing and/or reducing bronchial hyper-reactivity of the respiratory tract, thus resolving the phlogistic condition accompanying acute and subchronical inflammations of bronchial mucosa.

The compounds of the invention of formula (I) are prepared from thiazoles of formula (II)

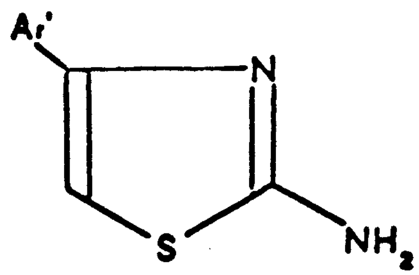

(II)

wherein **Ar'** has the same meanings as Ar, or is a group which can be converted into Ar by removing any protective groups present, by reacting them first with carbonyldiimidazole or, when X is S, thiocarbonyldiimidazole, or similar difunctional carbonylating agents, then with amines of formula (III):

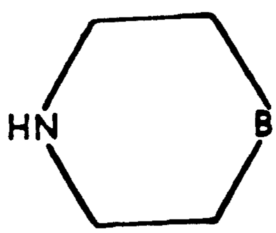

(III)

wherein B is as defined above.

The reaction of compound (II), with carbonyldiimidazole or its thioanalogue and amines (III) is generally carried out in anhydrous aprotic solvents, at temperatures ranging from room temperature to the reflux temperature of the reaction mixture. Suitable solvents are ethyl ether, tetrahydrofuran, halogenated hydrocarbons, dimethylsufoxide, dimethylformamide. Of course carbonyldiimidazole can be replaced by similar reagents, such as phosgene or thiophosgene.

Compounds of formula (II) are known or they can be prepared by reacting thiourea with haloacetophenones of formula (IV),

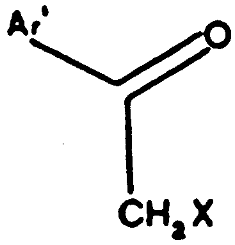

(IV)

wherein Ar' is as defined above and X is a chlorine, bromine or iodine atom, which compounds are known or can be prepared by widely known methods.

Compounds of formula (III) are widely known or have been described in WO 87/01706.

Compounds of formula (I), as mentioned above, have useful pharmacological properties, particularly for the treatment of bronchial hyper-reactivity.

Bronchial hyper-reactivity is a clinical symptom of asthma and it is believed to be a direct consequence of an abnormal and latent contractility and sensitivity of the bronchial mucosa.

Bronchial hyper-reactivity can cause acute crisis of asthma after physical practice, and/or after exposure to external stimuli such as the inhalation of fog, pollutants, allergens and autacoids.

The bronchial hyper-reactivity conditions may be simulated by an experimental model consisting in the PAF infusion (600 µg/l) in male guinea-pigs weighing 400-450 g, kept under forced ventilation under urethane and pancuronium bromide anesthesia.

PAF, which is one of the most important mediators involved in the inflammatory process of the airways, after infusion for 1 hour, causes an hyperreactivity reaction (bronchocostriction) to specific and different substances.

The activity of the compounds of the invention, in the considered pharmacological model, is shown by the prevention of the PAF-induced hyper-reactivity, measured as increase of the pulmonary insufflatory pressure (measured according to the modified procedure of Konzett and Rossler, Naun. Schmied. Arch. Exper. Pathol. Pharmacol. 191, 71, 1970).

The compounds of the invention, which are administered 10 minutes before the PAF administration in dosages which vary between 2 and 50 µg/Kg, demonstrate a protective action which lasts at least 4-6 hours and results in a reduction of the PAF-induced hyperreactivity. Such pharmacological effects are dose-related.

From what has been shown above it is clear that the compounds of the invention can be used in human therapy in the treatment of asthmatic and obstructive conditions of the respiratory tract, in the treatment of inflammatory phlogosis. In the intended therapeutic uses, the compounds of the invention will be administered in the form of pharmaceutical compositions which can be prepared with excipients and conventional techniques such as, for example, those described in Remington's Pharmaceutical Sciences Handbook, Mack Pub. Co., N.Y., USA, 17th ed., 1985, adapted for administration by intramuscular, intravenous, oral, aerosol and rectal methods.

The daily dose will depend on several factors such as the gravity of the pathology and the condition of the patient: it will normally consist of from 1 to 50 mg of a compound of formula I for a patient weighing 70 kg, one or more times a day.

The following Examples and Preparations further illustrate the invention.

The 2-amino-4-arylthiazoles (II) are either commercially available or are prepared by reaction of the suitable α-bromoaryl-methylketones with thiourea, as disclosed in the following preparations 1-4.

**PREPARATION 1**

Acetic anhydride (2.7 ml) is dropped into a solution of 2,3-dihydroxybenzoic acid (2 g) in 10 ml of pyridine, cooling to 0° C. After 6 hours at room temperature, the reaction mixture is poured into 15 ml of 1N HCl and it is repeatedly extracted with AcOEt (3x50 ml). The combined organic extracts are washed with water, dried over sodium sulfate and the solvent is evaporated off under reduced pressure. The resulting residue (3.5 g) is crystallized from diisopropyl ether to give 2.7 g of 2,3-diacetoxybenzoic acid, m.p. 151-153°.

**PREPARATION 2**

Carbonyldiimidazole (750 mg) is added to a solution of 2,3-diacetoxybenzoic acid (1 g) in 10 ml of anhydrous tetrahydrofuran, with stirring and under inert gas atmosphere. After one hour, the reaction mixture is added with 570 mg of dimethylaminopyridine and 670 mg of Meldrum acid. After 2 more hours, the reaction mixture is poured into 15 ml of 1N HCl and repeatedly extracted with AcOEt (3x30 ml). The combined organic extracts are washed with water, dried over sodium sulfate and solvent is evaporated off under reduce pressure. The resulting residue (1.7 g) is purified by silica gel chromatography (50 g, eluent 8/2 AcOEt/hexane), to obtain 1.4 g of 5-((2,3-diacetoxyphenyl)carbonyl)-2,2-dimethyl-4,6-dioxo-1,3-dioxane.

**PREPARATION 3**

Paratoluenesulfonic acid mono-hydrate (1.15 g) is added to a solution of 5-((2,3-diacetoxyphenyl)-carbonyl)-2,2-dimethyl-4,6-dioxo-1,3-dioxane (2 g) in 10 ml of acetonitrile. After 24 hours, the reaction mixture is poured into a $NaHCO_3$ saturated solution (20 ml) and repeatedly extracted with AcOEt (3x30 ml). The combined organic extracts are washed with water, dried over sodium sulfate and solvent is evaporated off under reduced pressure, to obtain 1.1 g of 2,3-diacetoxyacetophenone.

**PREPARATION 4**

Bromine (0.24 ml) is dropped into a solution of 2,3-diacetoxyacetophenone (1 g) in 10 ml of dioxane, under stirring and keeping temperature at 0-5 ° C. When dropping is over, the reaction mixture is warmed to room temperature and stirring is continued for one hour, then it is poured into a $NaHCO_3$ saturated solution (15 ml) and repeatedly extracted with AcOEt (3x30 ml). The combined organic extracts are washed with water, dried over sodium sulfate and solvent is evaporated off under reduced pressure, to give 1.2 g of diacetoxyphenacyl bromide.

Thiourea (360 mg) is added to a solution of 2,3-diacetoxyphenacyl bromide (1 g) in 10 ml of ethanol. After 2 hours, 950 mg of 2-amino-4-(2,3-diacetoxyphenyl)thiazole are separated by filtration.

Following the same procedure of preparation 1 to 4, 2-amino-4-(4-acetoxyphenyl)thiazole, 2-amino-4-(4-acetoxy-3,5-di-tert-butylphenyl)thiazole and 2-amino-4-(3,4-diacetoxyphenyl)thiazole are prepared.

**EXAMPLE 1**

Carbonyldiimidazole (920 mg) is added to a solution of 2-amino-4-(2,3-diacetoxyphenyl)thiazole (1.5 g) in 10 ml of anhydrous tetrahydrofuran. After 1 hour, N-(3,6-bis-diethylamino-pyridin-2-yl)piperazine (1.7 g) is added to the reaction mixture. After 2 hours at room temperature, the reaction mixture is poured into a $NaHCO_3$ saturated solution (15 ml) and repeatedly extracted with AcOEt (3x30 ml). The combined organic extracts are washed with water, dried over sodium sulfate and the solvent is evaporated under reduced pressure. The resulting residue (3 g) is purified by silica gel chromatography (90 g; eluent 95/5 AcOEt/MeOH), to obtain 2.5 g of N-(3,6-bis-diethylamino-pyridin-2-yl)-N'-(4-(2,3-diacetoxyphenyl)-thiazol-2-yl)aminocarbonyl)piperazine.

**EXAMPLE 2**

NaOH (0.37 ml, 35% aqueous solution) is added to a solution of N-(3,6-bis-diethylamino-pyridin-2-yl)-N'-(4-(2,3-diacetoxyphenyl)-thiazol-2-yl)aminocarbonyl)piperazine (2.5 g) in 10 ml of methanol. After one hour, the reaction mixture is poured into a 0.1N HCl solution (10 ml) and repeatedly extracted with AcOEt (3x30 ml). The combined organic extracts are washed with water, dried over sodium sulfate and solvent is evaporated off under reduced pressure, to give 2 g of N-(3,6-bis-diethylamino-pyridin-2-yl)-N'-(4-(2,3-dihydroxyphenyl)-thiazol-2-yl)aminocarbonyl)piperazine.

**EXAMPLE 3**

Following the procedures described in the above Examples, starting from thiazoles of Preparation 4 and the appropriate N-substituted piperazines, the following compounds of formula (I) are obtained:

N-(3,6-bis-diethylamino-pyridin-2-yl)-N'-(4-(4-hydroxyphenyl)thiazol-2-yl)aminocarbonyl)piperazine;

N-(3,6-bis-diethylamino-pyridin-2-yl)-N'-(4-(4-hydroxy-3,5-di-tert-butylphenyl)thiazol-2-yl)aminocarbonyl)-piperazine, m.p. of hydrochloride 177-180 ° C;

N-(3,6-bis-diethylamino-pyridin-2-yl)-N'-(4-(3,4-dihydroxyphenyl)thiazol-2-yl)aminocarbonyl)piperazine;

N-(2,6-bis(pyrrolidin-1-yl)pyrimidin-4-yl)-N'-(4-(4-hydroxyphenyl)thiazol-2-yl)aminocarbonyl)piperazine;

N-(2,6-bis(pyrrolidin-1-yl)pyrimidin-4-yl)-N'-(4-(4-hydroxy-3,5-di-tert-butylphenyl)thiazol-2-yl)aminocarbonyl)-piperazine;
N-[4,6-bis(pyrrolidin-1-yl)pyrimidin-2-yl)-N'-(4-(4-hydroxy-3,5-di-tert-butylphenyl)thiazol-2-yl)aminocarbonyl)-piperazine, m.p. 162-165° C;
N-(2,6-bis(pyrrolidin-1-yl)pyrimidin-4-yl)-N'-(4-(3,4-dihydroxyphenyl)thiazol-2-yl)aminocarbonyl)piperazine;
N-(2,6-bis(pyrrolidin-1-yl)pyrimidin-4-yl)-N'-(4-(2,3-dihydroxyphenyl)-thiazol-2-yl)aminocarbonyl)piperazine, m.p. 172-174° C;
N-(4,6-bis(pyrrolidin-1-yl)-1,3,5-triazin-2-yl)-N'-(4-(4-hydroxyphenyl)thiazol-2-yl)aminocarbonyl)piperazine;
N-(4,6-bis(pyrrolidin-1-yl)-1,3,5-triazin-2-yl)-N'-(4-(4-hydroxy-3,5-di-tert-butylphenyl)thiazol-2-yl)-aminocarbonyl)piperazine;
N-(4,6-bis(pyrrolidin-1-yl)-1,3,5-triazin-2-yl)-N'-(4-(3,4-dihydroxyphenyl)thiazol-2-yl)aminocarbonyl)piperazine;
N-(4,6-bis(pyrrolidin-1-yl)-1,3,5-triazin-2-yl)-N'-(4-(2,3-dihydroxyphenyl)thiazol-2-yl)aminocarbonyl)piperazine.

## EXAMPLE 4

Following the procedures described in the above Examples, starting from acetophenone and the appropriate N-substituted piperazines, the following N,N'-disubstituted piperazines are obtained:
N-(4,6-bis(pyrrolidin-1-yl)-1,3,5-triazin-2-yl)-N'-(4-phenylthiazol-2-yl)-aminocarbonyl)piperazine, m.p. 155-159° C;
N-(3,6-bis-diethylamino-pyridin-2-yl)-N'-(4-phenylthiazol-2-yl)aminocarbonyl)piperazine, m.p. of hydrochloride 159-162° C;
N-(2,6-bis(pyrrolidin-1-yl)pyrimidin-4-yl)-N'-(4-phenylthiazol-2-yl)aminocarbonyl)piperazine, m.p. 147-148° C;
N-(4,6-bis(pyrrolidin-1-yl)pyrimidin-2-yl)-N'-((4-phenylthiazol-2-yl)aminocarbonyl)piperazine,
N-methyl-N'-((4-phenylthiazol-2-yl)aminocarbonyl)piperazine, m.p.151-152° C,
N-(bis(4-fluorophenyl)methyl)-N'-((4-phenylthiazol-2-yl)aminocarbonyl)piperazine, m.p. of hydrochloride 166-169° C,
N-(4-fluorophenyl)-N'-((4-phenylthiazol-2-yl)aminocarbonyl)piperazine, m.p. of hydrochloride 232-234° C,
N-(bis(4-chlorophenyl)methyl)-N'-((4-phenylthiazol-2-yl)aminocarbonyl)piperazine,
N-(4-chlorophenyl)-N'-((4-phenylthiazol-2-yl)aminocarbonyl)piperazine,
N-(bis(phenyl)methyl)-N'-((4-phenylthiazol-2-yl)aminocarbonyl)piperazine, m.p. of hydrochloride 187-189° C,
N-(3,6-bis(diethylamino)pyridin-2-yl)-N'-((4-(4-methoxyphenyl)thiazol-2-yl)aminocarbonyl)piperazine, m.p. of hydrobromide 150-153° C,
N-(2,6-bis(pyrrolidin-1-yl)pyrimidin-4-yl)-N'-((4-(4-methoxyphenyl)thiazol-2-yl)aminocarbonyl)piperazine,
N-(4,6-bis(pyrrolidin-1-yl)-1,3,5-triazin-2-yl)-N'-((4-(4-methoxyphenyl)thiazol-2-yl)aminocarbonyl)piperazine,
N-methyl-N'-((4-(4-methoxyphenyl)thiazol-2-yl)aminocarbonyl)piperazine, m.p. 140-143° C,
N-(bis(4-fluorophenyl)methyl)-N'-((4-(4-methoxyphenyl)thiazol-2-yl)aminocarbonyl)piperazine,
N-(4-fluorophenyl)-N'-((4-(4-methoxyphenyl)thiazol-2-yl)aminocarbonyl)piperazine, m.p. of hydrobromide 210-214° C,
N-(bis(phenyl)methyl)-N'-((4-(4-methoxyphenyl)thiazol-2-yl)aminocarbonyl)piperazine, m.p. 162-165° C,
N-(2,6-bis(pyrrolidin-1-yl)pyrimidin-4-yl)-N'-((4-(3-methoxyphenyl)thiazol-2-yl)aminocarbonyl)piperazine,
N-(4,6-bis(pyrrolidin-1-yl)-1,3,5-triazin-2-yl)-N'-((4-(2-methoxyphenyl)thiazol-2-yl)aminocarbonyl)piperazine, m.p. 171-173° C,
N-methyl-N'-((4-(3-methoxyphenyl)thiazol-2-yl)aminocarbonyl)piperazine,
N-(3,6-bis(diethylamino)pyridin-2-yl)-N'-((4-(4-clorophenyl)thiazol-2-yl)aminocarbonyl)piperazine, m.p. of hydrochloride 147-150° C,
N-(2,6-bis(pyrrolidin-1-yl)pyrimidin-4-yl)-N'-((4-(4-clorophenyl)thiazol-2-yl)aminocarbonyl)piperazine,
N-(4,6-bis(pyrrolidin-1-yl)pyrimidin-2-yl)-N'-((4-(4-chlorophenyl)thiazol-2-yl)aminocarbonyl)piperazine,
N-(4,6-bis(pyrrolidin-1-yl)-1,3,5-triazin-2-yl)-N'-((4-(4-chlorophenyl)thiazol-2-yl)aminocarbonyl)piperazine,
N-methyl-N'-((4-(4-clorophenyl)thiazol-2-yl)aminocarbonyl)piperazine, m.p. 137-139° C,
N-(bis(4-fluorophenyl)methyl)-N'-((4-(4-chlorophenyl)thiazol-2-yl)aminocarbonyl)piperazine,
N-(4-fluorophenyl)-N'-((4-(4-chlorophenyl)thiazol-2-yl)aminocarbonyl)piperazine, m.p. 164-168° C,
N-(4,6-bis(pyrrolidin-1-yl)-1,3,5-triazin-2-yl)-N'-((4-(3-chlorophenyl)thiazol-2-yl)aminocarbonyl)piperazine,
N-(2,6-bis(pyrrolidin-1-yl)pyrimidin-4-yl)-N'-((4-(2-chlorophenyl)thiazol-2-yl)aminocarbonyl)piperazine,
N-methyl-N'-((4-(2-chlorophenyl)thiazol-2-yl)aminocarbonyl)piperazine, m.p. 146-150° C,
N-(3,6-bis(diethylamino)pyridin-2-yl)-N'-((4-(4-fluorophenyl)thiazol-2-yl)aminocarbonyl)piperazine,
N-(2,6-bis(pyrrolidin-1-yl)pyrimidin-4-yl)-N'-((4-(4-fluorophenyl)thiazol-2-yl)aminocarbonyl)piperazine, m.p. 174-177° C,
N-(4,6-bis(pyrrolidin-1-yl)-1,3,5-triazin-2-yl)-N'-((4-(4-fluorophenyl)thiazol-2-yl)aminocarbonyl)piperazine,

N-methyl-N'-((4-(4-fluorophenyl)thiazol-2-yl)aminocarbonyl)piperazine, m.p. 150-153 ° C,
N-(bis(4-fluorophenyl)methyl)-N'-((4-(4-fluorophenyl)thiazol-2-yl)aminocarbonyl)piperazine,
N-(2,6-bis(pyrrolidin-1-yl)pyrimidin-4-yl)-N'-((4-(2-fluorophenyl)thiazol-2-yl)aminocarbonyl)piperazine,
N-(2,6-bis(pyrrolidin-1-yl)pyrimidin-4-yl)-N'-((4-(4-bromophenyl)thiazol-2-yl)aminocarbonyl)piperazine,
N-(4,6-bis(pyrrolidin-1-yl)-1,3,5-triazin-2-yl)-N'-((4-(4-bromophenyl)thiazol-2-yl)aminocarbonyl)piperazine,
N-methyl-N'-((4-(4-bromophenyl)thiazol-2-yl)aminocarbonyl)piperazine, m.p. of hydrobromide 196-199 ° C,
N-(4-fluorophenyl)-N'-((4-(4-bromophenyl)thiazol-2-yl)aminocarbonyl)piperazine, m.p. of hydrobromide 236-241 ° C,
N-(4,6-bis(pyrrolidin-1-yl)-1,3,5-triazin-2-yl)-N'-((4-(3-bromophenyl)thiazol-2-yl)aminocarbonyl)piperazine,
N-(4-fluorophenyl)-N'-((4-(3-bromophenyl)thiazol-2-yl)aminocarbonyl)piperazine,
N-(3,6-bis(diethylamino)pyridin-2-yl)-N'-((4-(4-cyanophenyl)thiazol-2-yl)aminocarbonyl)piperazine,
N-(2,6-bis(pyrrolidin-1-yl)pyrimidin-4-yl)-N'-((4-(4-cyanophenyl)thiazol-2-yl)aminocarbonyl)piperazine,
N-(4,6-bis(pyrrolidin-1-yl)-1,3,5-triazin-2-yl)-N'-((4-(4-cyanophenyl)thiazol-2-yl)aminocarbonyl)piperazine,
N-methyl-N'-((4-(4-cyanophenyl)thiazol-2-yl)aminocarbonyl)piperazine,
N-(bis(4-chlorophenyl)methyl)-N'-((4-(4-cyanophenyl)thiazol-2-yl)aminocarbonyl)piperazine, m.p. 183-185 ° C,
N-(bis(4-fluorophenyl)methyl)-N'-((4-(4-cyanophenyl)thiazol-2-yl)aminocarbonyl)piperazine,
N-(4,6-bis(pyrrolidin-1-yl)-1,3,5-triazin-2-yl)-N'-((4-(3-cyanophenyl)thiazol-2-yl)aminocarbonyl)piperazine,
N-(2,6-bis(pyrrolidin-1-yl)pyrimidin-4-yl)-N'-((4-(4-nitrophenyl)thiazol-2-yl)aminocarbonyl)piperazine,
N-(4,6-bis(pyrrolidin-1-yl)-1,3,5-triazin-2-yl)-N'-((4-(4-nitrophenyl)thiazol-2-yl)aminocarbonyl)piperazine,
N-methyl-N'-((4-(4-nitrophenyl)thiazol-2-yl)aminocarbonyl)piperazine, m.p. 177-181 ° C,
N-(4-fluorophenyl)-N'-((4-(4-nitrophenyl)thiazol-2-yl)aminocarbonyl)piperazine,
N-(4-chlorophenyl)-N'-((4-(4-nitrophenyl)thiazol-2-yl)aminocarbonyl)piperazine,
N-(2,6-bis(pyrrolidin-1-yl)pyrimidin-4-yl)-N'-((4-(3-nitrophenyl)thiazol-2-yl)aminocarbonyl)piperazine,
N-methyl-N'-((4-(3-nitrophenyl)thiazol-2-yl)aminocarbonyl)piperazine,
N-(3,6-bis(diethylamino)pyridin-2-yl)-N'-((4-(4-phenylsulphonylphenyl)thiazol-2-yl)aminocarbonyl)piperazine, m.p. of hydrobromide 161-164 ° C,
N-(2,6-bis(pyrrolidin-1-yl)pyrimidin-4-yl)-N'-((4-(4-phenylsulphonylphenyl)thiazol-2-yl)aminocarbonyl)-piperazine,
N-(4,6-bis(pyrrolidin-1-yl)pyrimidin-2-yl)-N'-((4-(4-phenylsulphonylphenyl)thiazol-2-yl)aminocarbonyl)-piperazine,
N-(4,6-bis(pyrrolidin-1-yl)-1,3,5-triazin-2-yl)-N'-((4-(4-phenylsulphonylphenyl)thiazol-2-yl)aminocarbonyl)-piperazine,
N-methyl-N'-((4-(4-phenylsulphonylphenyl)thiazol-2-yl)aminocarbonyl)piperazine, m.p. 169-171 ° C,
N-(bis(4-fluorophenyl)methyl)-N'-((4-(4-phenylsulphonylphenyl)thiazol-2-yl)aminocarbonyl)piperazine,
N-(4,6-bis(pyrrolidin-1-yl)-1,3,5-triazin-2-yl)-N'-((4-(3-phenylsulphonylphenyl)thiazol-2-yl)aminocarbonyl)-piperazine,
N-methyl-N'-((4-(3-phenylsulphonylphenyl)thiazol-2-yl)aminocarbonyl)piperazine,
N-(2,6-bis(pyrrolidin-1-yl)pyrimidin-4-yl)-N'-((4-(4-(2'-chlorophenylsulphonyl)phenyl)thiazol-2-yl)-aminocarbonyl)piperazine,
N-(bis(4-fluorophenyl)methyl)-N'-((4-(4-(2'-chlorophenylsulphonyl)phenyl)thiazol-2-yl)aminocarbonyl)-piperazine, m.p. 190-195 ° C,
N-(2,6-bis(pyrrolidin-1-yl)pyrimidin-4-yl)-N'-((4-(3-(2'-chlorophenylsulphonyl)phenyl)thiazol-2-yl)-aminocarbonyl)piperazine,
N-(3,6-bis(diethylamino)pyridin-2-yl)-N'-((4-((4-methylsulfonylamino-3-phenoxy)phenyl)thiazol-2-yl)-aminocarbonyl)piperazine, m.p. of hydrobromide 200-205 ° C,
N-(bis(phenyl)methyl)-N'-((4-((4-methylsulfonylamino-3-phenoxy)phenyl)thiazol-2-yl)aminocarbonyl)-piperazine, m.p. 162-165 ° C,
N-methyl-N'-((4-((4-methylsulfonylamino-3-phenoxy)phenyl)thiazol-2-yl)aminocarbonyl)piperazine,
N-(3,6-bis(diethylamino)pyridin-2-yl)-N'-((4-(4-phenyltiophenyl)thiazol-2-yl)aminocarbonyl)piperazine,
N-(2,6-bis(pyrrolidin-1-yl)pyrimidin-4-yl)-N'-((4-(4-phenyltiophenyl)thiazol-2-yl)aminocarbonyl)piperazine,
N-methyl-N'-((4-(4-phenyltiophenyl)thiazol-2-yl)aminocarbonyl)piperazine, m.p. 158-160 ° C,
N-(2,6-bis(pyrrolidin-1-yl)pyrimidin-4-yl)-N'-((4-(4-aminophenyl)thiazol-2-yl)aminocarbonyl)piperazine,
N-(4,6-bis(pyrrolidin-1-yl)-1,3,5-triazin-2-yl)-N'-((4-(4-aminophenyl)thiazol-2-yl)aminocarbonyl)piperazine,
N-methyl-N'-((4-(4-aminophenyl)thiazol-2-yl)aminocarbonyl)piperazine, m.p. 163-165 ° C,
N-(bis(4-fluorophenyl)methyl)-N'-((4-(4-aminophenyl)thiazol-2-yl)aminocarbonyl)piperazine,
N-(2,6-bis(pyrrolidin-1-yl)pyrimidin-4-yl)-N'-((4-(3-aminophenyl)thiazol-2-yl)aminocarbonyl)piperazine,
N-(4,6-bis(pyrrolidin-1-yl)-1,3,5-triazin-2-yl)-N'-((4-(2-aminophenyl)thiazol-2-yl)aminocarbonyl)piperazine,
N-(3,6-bis(diethylamino)pyridin-2-yl)-N'-((4-(4-((pyrrolidin-1'-yl)methyl)phenyl)thiazol-2-yl)aminocarbonyl)-

piperazine, m.p. of hydrochloride 221-225 ° C,
N-(2,6-bis(pyrrolidin-1-yl)pyrimidin-4-yl)-N'-((4-(4-((pyrrolidin-1'-yl)methyl)phenyl)thiazol-2-yl)aminocarbonyl)-piperazine,
N-(4,6-bis(pyrrolidin-1-yl)-1,3,5-triazin-2-yl)-N'-((4-(4-((pyrrolidin-1'-yl)methyl)phenyl)thiazol-2-yl)-aminocarbonyl)piperazine,
N-methyl-N'-((4-(4-((pyrrolidin-1'-yl)methyl)phenyl)thiazol-2-yl)aminocarbonyl)piperazine, m.p. of hydrochloride 218-220 ° C,
N-(bis(4-fluorophenyl)methyl)-N'-((4-(4-((pyrrolidin-1'-yl)methyl)phenyl)thiazol-2-yl)aminocarbonyl)piperazine,
N-(4-fluorophenyl)-N'-((4-(4-((pyrrolidin-1'-yl)methyl)phenyl)thiazol-2-yl)aminocarbonyl)piperazine, m.p. of hydrochloride 242-246 ° C,
N-(4-chlorophenyl)-N'-((4-(4-((pyrrolidin-1'-yl)methyl)phenyl)thiazol-2-yl)aminocarbonyl)piperazine,
N-(2,6-bis(pyrrolidin-1-yl)pyrimidin-4-yl)-N'-((4-(3-((pyrrolidin-1'-yl)methyl)phenyl)thiazol-2-yl)aminocarbonyl)-piperazine,
N-(2,6-bis(pyrrolidin-1-yl)pyrimidin-4-yl)-N'-((4-(4-methoxycarbonylphenyl)thiazol-2-yl)aminocarbonyl)-piperazine,
N-(4,6-bis(pyrrolidin-1-yl)-1,3,5-triazin-2-yl)-N'-((4-(4-methoxycarbonylphenyl)thiazol-2-yl)aminocarbonyl)-piperazine,
N-methyl-N'-((4-(4-methoxycarbonylphenyl)thiazol-2-yl)aminocarbonyl)piperazine, m.p. 156-157 ° C,
N-(4,6-bis(pyrrolidin-1-yl)-1,3,5-triazin-2-yl)-N'-((4-(3-methoxycarbonylphenyl)thiazol-2-yl)aminocarbonyl)-piperazine,
N-(2,6-bis(pyrrolidin-1-yl)pyrimidin-4-yl)-N'-((4-(pyridin-3-yl)thiazol-2-yl)aminocarbonyl)piperazine,
N-(4,6-bis(pyrrolidin-1-yl)-1,3,5-triazin-2-yl)-N'-((4-(pyridin-3-yl)thiazol-2-yl)aminocarbonyl)piperazine,
N-methyl-N'-((4-(pyridin-3-yl)thiazol-2-yl)aminocarbonyl)piperazine, m.p. of hydrochloride 200-203 ° C,
N-(2,6-bis(pyrrolidin-1-yl)pyrimidin-4-yl)-N'-((4-(3,4-dichlorophenyl)thiazol-2-yl)aminocarbonyl)piperazine,
N-(4,6-bis(pyrrolidin-1-yl)-1,3,5-triazin-2-yl)-N'-((4-(3,4-dichlorophenyl)thiazol-2-yl)aminocarbonyl)piperazine,
N-methyl-N'-((4-(3,4-dichlorophenyl)thiazol-2-yl)aminocarbonyl)piperazine, m.p. 148-151 ° C,
N-(2,6-bis(pyrrolidin-1-yl)pyrimidin-4-yl)-N'-((4-(4-trifluoromethylphenyl)thiazol-2-yl)aminocarbonyl)piperazine,
N-(4,6-bis(pyrrolidin-1-yl)-1,3,5-triazin-2-yl)-N'-((4-(4-trifluoromethylphenyl)thiazol-2-yl)aminocarbonyl)-piperazine,
N-methyl-N'-((4-(4-trifluoromethylphenyl)thiazol-2-yl)aminocarbonyl)piperazine, m.p. 173-174 ° C,
N-(2,6-bis(pyrrolidin-1-yl)pyrimidin-4-yl)-N'-((4-(3-trifluoromethylphenyl)thiazol-2-yl)aminocarbonyl)piperazine,
N-(4,6-bis(pyrrolidin-1-yl)-1,3,5-triazin-2-yl)-N'-((4-(3-trifluoromethylphenyl)thiazol-2-yl)aminocarbonyl)-piperazine,
N-methyl-N'-((4-(3-trifluoromethylphenyl)thiazol-2-yl)aminocarbonyl)piperazine.

## EXAMPLE 5

By substituting, in the procedure of Example 1, thiocarbonyl diimidazole to carbonyldiimidazole, the following thioureas are prepared starting from the suitable 2-amino-4-arylthiazoles and from the suitable N-substituted-piperazines:
N-(3,6-bis(diethylamino)pyridin-2-yl)-N'-((4-phenylthiazol-2-yl)aminothiocarbonyl)piperazine,
N-(2,6-bis(pyrrolidin-1-yl)pyrimidin-4-yl)-N'-((4-phenylthiazol-2-yl)aminothiocarbonyl)piperazine, m.p. 207-209 ° C,
N-(4,6-bis(pyrrolidin-1-yl)-1,3,5-triazin-2-yl)-N'-((4-phenylthiazol-2-yl)aminothiocarbonyl)piperazine,
N-methyl-N'-((4-phenylthiazol-2-yl)aminothiocarbonyl)piperazine,
N-(bis(4-fluorophenyl)methyl)-N'-((4-phenylthiazol-2-yl)aminothiocarbonyl)piperazine,
N-(4-fluorophenyl)-N'-((4-phenylthiazol-2-yl)aminothiocarbonyl)piperazine, m.p. 216-219 ° C,
N-(bis(4-chlorophenyl)methyl)-N'-((4-phenylthiazol-2-yl)aminothiocarbonyl)piperazine,
N-(4-chlorophenyl)-N'-((4-phenylthiazol-2-yl)aminothiocarbonyl)piperazine,
N-(bis(phenyl)methyl)-N'-((4-phenylthiazol-2-yl)aminothiocarbonyl)piperazine,
N-(3,6-bis(diethylamino)pyridin-2-yl)-N'-((4-(4-methoxyphenyl)thiazol-2-yl)aminothiocarbonyl)piperazine,
N-(2,6-bis(pyrrolidin-1-yl)pyrimidin-4-yl)-N'-((4-(4-methoxyphenyl)thiazol-2-yl)aminothiocarbonyl)piperazine,
N-(4,6-bis(pyrrolidin-1-yl)-1,3,5-triazin-2-yl)-N'-((4-(4-methoxyphenyl)thiazol-2-yl)aminothiocarbonyl)-piperazine,
N-methyl-N'-((4-(4-methoxyphenyl)thiazol-2-yl)aminothiocarbonyl)piperazine,
N-(bis(4-fluorophenyl)methyl)-N'-((4-(4-methoxyphenyl)thiazol-2-yl)aminothiocarbonyl)piperazine,
N-(4-fluorophenyl)-N'-((4-(4-methoxyphenyl)thiazol-2-yl)aminothiocarbonyl)piperazine, m.p. of hydrobromide 240-242 ° C,

N-(bis(phenyl)methyl)-N'-((4-(4-methoxyphenyl)thiazol-2-yl)aminothiocarbonyl)piperazine,
N-(2,6-bis(pyrrolidin-1-yl)pyrimidin-4-yl)-N'-((4-(3-methoxyphenyl)thiazol-2-yl)aminothiocarbonyl)piperazine,
N-(4,6-bis(pyrrolidin-1-yl)-1,3,5-triazin-2-yl)-N'-((4-(2-methoxyphenyl)thiazol-2-yl)aminothiocarbonyl)-piperazine,
N-methyl-N'-((4-(3-methoxyphenyl)thiazol-2-yl)aminothiocarbonyl)piperazine,
N-(3,6-bis(diethylamino)pyridin-2-yl)-N'-((4-(4-chlorophenyl)thiazol-2-yl)aminothiocarbonyl)piperazine, m.p. 170-174° C,
N-(2,6-bis(pyrrolidin-1-yl)pyrimidin-4-yl)-N'-((4-(4-chlorophenyl)thiazol-2-yl)aminothiocarbonyl)piperazine,
N-(4,6-bis(pyrrolidin-1-yl)pyrimidin-2-yl)-N'-((4-(4-chlorophenyl)thiazol-2-yl)aminothiocarbonyl)piperazine,
N-(4,6-bis(pyrrolidin-1-yl)-1,3,5-triazin-2-yl)-N'-((4-(4-chlorophenyl)thiazol-2-yl)aminothiocarbonyl)piperazine,
N-methyl-N'-((4-(4-chlorophenyl)thiazol-2-yl)aminothiocarbonyl)piperazine,
N-(bis(4-fluorophenyl)methyl)-N'-((4-(4-chlorophenyl)thiazol-2-yl)aminothiocarbonyl)piperazine,
N-(4-fluorophenyl)-N'-((4-(4-chlorophenyl)thiazol-2-yl)aminothiocarbonyl)piperazine, m.p. 199-202° C,
N-(4,6-bis(pyrrolidin-1-yl)-1,3,5-triazin-2-yl)-N'-((4-(3-chlorophenyl)thiazol-2-yl)aminothiocarbonyl)piperazine,
N-(2,6-bis(pyrrolidin-1-yl)pyrimidin-4-yl)-N'-((4-(2-chlorophenyl)thiazol-2-yl)aminothiocarbonyl)piperazine,
N-methyl-N'-((4-(2-chlorophenyl)thiazol-2-yl)aminothiocarbonyl)piperazine,
N-(3,6-bis(diethylamino)pyridin-2-yl)-N'-((4-(4-fluorophenyl)thiazol-2-yl)aminothiocarbonyl)piperazine,
N-(2,6-bis(pyrrolidin-1-yl)pyrimidin-4-yl)-N'-((4-(4-fluorophenyl)thiazol-2-yl)aminothiocarbonyl)piperazine,
N-(4,6-bis(pyrrolidin-1-yl)-1,3,5-triazin-2-yl)-N'-((4-(4-fluorophenyl)thiazol-2-yl)aminothiocarbonyl)piperazine,
N-methyl-N'-((4-(4-fluorophenyl)thiazol-2-yl)aminothiocarbonyl)piperazine, m.p. 188-192° C,
N-(bis(4-fluorophenyl)methyl)-N'-((4-(4-fluorophenyl)thiazol-2-yl)aminothiocarbonyl)piperazine,
N-(2,6-bis(pyrrolidin-1-yl)pyrimidin-4-yl)-N'-((4-(2-fluorophenyl)thiazol-2-yl)aminothiocarbonyl)piperazine,
N-(2,6-bis(pyrrolidin-1-yl)pyrimidin-4-yl)-N'-((4-(4-bromophenyl)thiazol-2-yl)aminothiocarbonyl)piperazine,
N-(4,6-bis(pyrrolidin-1-yl)-1,3,5-triazin-2-yl)-N'-((4-(4-bromophenyl)thiazol-2-yl)aminothiocarbonyl)piperazine,
N-methyl-N'-((4-(4-bromophenyl)thiazol-2-yl)aminothiocarbonyl)piperazine, m.p. of hydrobromide 233-239° C,
N-(4-fluorophenyl)-N'-((4-(4-bromophenyl)thiazol-2-yl)aminothiocarbonyl)piperazine,
N-(4,6-bis(pyrrolidin-1-yl)-1,3,5-triazin-2-yl)-N'-((4-(3-bromophenyl)thiazol-2-yl)aminothiocarbonyl)piperazine,
N-(4-fluorophenyl)-N'-((4-(3-bromophenyl)thiazol-2-yl)aminothiocarbonyl)piperazine,
N-(3,6-bis(diethylamino)pyridin-2-yl)-N'-((4-(4-cyanophenyl)thiazol-2-yl)aminothiocarbonyl)piperazine,
N-(2,6-bis(pyrrolidin-1-yl)pyrimidin-4-yl)-N'-((4-(4-cyanophenyl)thiazol-2-yl)aminothiocarbonyl)piperazine,
N-(4,6-bis(pyrrolidin-1-yl)-1,3,5-triazin-2-yl)-N'-((4-(4-cyanophenyl)thiazol-2-yl)aminothiocarbonyl)piperazine,
N-methyl-N'-((4-(4-cyanophenyl)thiazol-2-yl)aminothiocarbonyl)piperazine,
N-(bis(4-chlorophenyl)methyl)-N'-((4-(4-cyanophenyl)thiazol-2-yl)aminothiocarbonyl)piperazine,
N-(bis(4-fluorophenyl)methyl)-N'-((4-(4-cyanophenyl)thiazol-2-yl)aminothiocarbonyl)piperazine,
N-(4,6-bis(pyrrolidin-1-yl)-1,3,5-triazin-2-yl)-N'-((4-(3-cyanophenyl)thiazol-2-yl)aminothiocarbonyl)piperazine,
N-(2,6-bis(pyrrolidin-1-yl)pyrimidin-4-yl)-N'-((4-(4-nitrophenyl)thiazol-2-yl)aminothiocarbonyl)piperazine,
N-(4,6-bis(pyrrolidin-1-yl)-1,3,5-triazin-2-yl)-N'-((4-(4-nitrophenyl)thiazol-2-yl)aminothiocarbonyl)piperazine,
N-methyl-N'-((4-(4-nitrophenyl)thiazol-2-yl)aminothiocarbonyl)piperazine, m.p. 211-214° C,
N-(4-fluorophenyl)-N'-((4-(4-nitrophenyl)thiazol-2-yl)aminothiocarbonyl)piperazine,
N-(4-chlorophenyl)-N'-((4-(4-nitrophenyl)thiazol-2-yl)aminothiocarbonyl)piperazine,
N-(2,6-bis(pyrrolidin-1-yl)pyrimidin-4-yl)-N'-((4-(3-nitrophenyl)thiazol-2-yl)aminothiocarbonyl)piperazine,
N-methyl-N'-((4-(3-nitrophenyl)thiazol-2-yl)aminothiocarbonyl)piperazine,
N-(3,6-bis(diethylamino)pyridin-2-yl)-N'-((4-(4-phenylsulphonylphenyl)thiazol-2-yl)aminothiocarbonyl)-piperazine, m.p. of hydrobromide 202-205° C,
N-(2,6-bis(pyrrolidin-1-yl)pyrimidin-4-yl)-N'-((4-(4-phenylsulphonylphenyl)thiazol-2-yl)aminothiocarbonyl)-piperazine,
N-(4,6-bis(pyrrolidin-1-yl)pyrimidin-2-yl)-N'-((4-(4-phenylsulphonylphenyl)thiazol-2-yl)aminothiocarbonyl)-piperazine,
N-(4,6-bis(pyrrolidin-1-yl)-1,3,5-triazin-2-yl)-N'-((4-(4-phenylsulphonylphenyl)thiazol-2-yl)aminothiocarbonyl)-piperazine,
N-methyl-N'-((4-(4-phenylsulphonylphenyl)thiazol-2-yl)aminothiocarbonyl)piperazine, m.p. 193-197° C,
N-(bis(4-fluorophenyl)methyl)-N'-((4-(4-phenylsulphonylphenyl)thiazol-2-yl)aminothiocarbonyl)piperazine,
N-(4,6-bis(pyrrolidin-1-yl)-1,3,5-triazin-2-yl)-N'-((4-(3-phenylsulphonylphenyl)thiazol-2-yl)aminothiocarbonyl)-piperazine,
N-methyl-N'-((4-(3-phenylsulphonylphenyl)thiazol-2-yl)aminothiocarbonyl)piperazine,
N-(2,6-bis(pyrrolidin-1-yl)pyrimidin-4-yl)-N'-((4-(4-(2'-chlorophenylsulphonyl)phenyl)thiazol-2-yl)-aminothiocarbonyl)piperazine,

N-(bis(4-fluorophenyl)methyl)-N'-((4-(4-(2'-chlorophenylsulphonyl)phenyl)thiazol-2-yl)aminothiocarbonyl)-piperazine,
N-(2,6-bis(pyrrolidin-1-yl)pyrimidin-4-yl)-N'-((4-(3-(2'-chlorophenylsulphonyl)phenyl)thiazol-2-yl)-aminothiocarbonyl)piperazine,
N-(3,6-bis(diethylamino)pyridin-2-yl)-N'-((4-((4-methylsulfonylamino-3-phenoxy)phenyl)thiazol-2-yl)-aminothiocarbonyl)piperazine,
N-(bis(phenyl)methyl)-N'-((4-((4-methylsulfonyl)amino-3-phenoxy)phenyl)thiazol-2-yl)aminothiocarbonyl)-piperazine, m.p. of hydrobromide 227-230 ° C,
N-methyl-N'-((4-((4-methylsulfonylamino-3-phenoxy)phenyl)thiazol-2-yl)aminothiocarbonyl)piperazine, m.p. of hydrobromide 216-221 ° C,
N-(3,6-bis(diethylamino)pyridin-2-yl)-N'-((4-(4-phenylthiophenyl)thiazol-2-yl)aminothiocarbonyl)piperazine,
N-(2,6-bis(pyrrolidin-1-yl)pyrimidin-4-yl)-N'-((4-(4-phenylthiophenyl)thiazol-2-yl)aminothiocarbonyl)piperazine,
N-methyl-N'-((4-(4-phenylthiophenyl)thiazol-2-yl)aminothiocarbonyl)piperazine, m.p. 206-208 ° C,
N-(2,6-bis(pyrrolidin-1-yl)pyrimidin-4-yl)-N'-((4-(4-aminophenyl)thiazol-2-yl)aminothiocarbonyl)piperazine,
N-(4,6-bis(pyrrolidin-1-yl)-1,3,5-triazin-2-yl)-N'-((4-(4-aminophenyl)thiazol-2-yl)aminothiocarbonyl)piperazine,
N-methyl-N'-((4-(4-aminophenyl)thiazol-2-yl)aminothiocarbonyl)piperazine,
N-(bis(4-fluorophenyl)methyl)-N'-((4-(4-aminophenyl)thiazol-2-yl)aminothiocarbonyl)piperazine,
N-(2,6-bis(pyrrolidin-1-yl)pyrimidin-4-yl)-N'-((4-(3-aminophenyl)thiazol-2-yl)aminothiocarbonyl)piperazine,
N-(4,6-bis(pyrrolidin-1-yl)-1,3,5-triazin-2-yl)-N'-((4-(2-aminophenyl)thiazol-2-yl)aminothiocarbonyl)piperazine,
N-(3,6-bis(diethylamino)pyridin-2-yl)-N'-((4-(4-((pyrrolidin-1'-yl)methyl)phenyl)thiazol-2-yl)ami nothiocarbonyl)piperazine,
N-(2,6-bis(pyrrolidin-1-yl)pyrimidin-4-yl)-N'-((4-(4-((pyrrolidin-1'-yl)methyl)phenyl)thiazol-2-yl)-aminothiocarbonyl)piperazine,
N-(4,6-bis(pyrrolidin-1-yl)-1,3,5-triazin-2-yl)-N'-((4-(4-((pyrrolidin-1'-yl)methyl)phenyl)thiazol-2-yl)-aminothiocarbonyl)piperazine,
N-methyl-N'-((4-(4-((pyrrolidin-1'-yl)methyl)phenyl)thiazol-2-yl)aminothiocarbonyl)piperazine, m.p. of hydrochloride 254-258 ° C,
N-(bis(4-fluorophenyl)methyl)-N'-((4-(4-((pyrrolidin-1'-yl)methyl)phenyl)thiazol-2-yl)aminothiocarbonyl)-piperazine,
N-(4-fluorophenyl)-N'-((4-(4-((pyrrolidin-1'-yl)methyl)phenyl)thiazol-2-yl)aminothiocarbonyl)piperazine,
N-(4-chlorophenyl)-N'-((4-(4-((pyrrolidin-1'-yl)methyl)phenyl)thiazol-2-yl)aminothiocarbonyl)piperazine,
N-(2,6-bis(pyrrolidin-1-yl)pyrimidin-4-yl)-N'-((4-(3-((pyrrolidin-1'-yl)methyl)phenyl)thiazol-2-yl)-aminothiocarbonyl)piperazine,
N-(2,6-bis(pyrrolidin-1-yl)pyrimidin-4-yl)-N'-((4-(4-methoxycarbonylphenyl)thiazol-2-yl)aminothiocarbonyl)-piperazine,
N-(4,6-bis(pyrrolidin-1-yl)-1,3,5-triazin-2-yl)-N'-((4-(4-methoxycarbonylphenyl)thiazol-2-yl)aminothiocarbonyl)-piperazine,
N-methyl-N'-((4-(4-methoxycarbonylphenyl)thiazol-2-yl)aminothiocarbonyl)piperazine, m.p. 188-189 ° C,
N-(4,6-bis(pyrrolidin-1-yl)-1,3,5-triazin-2-yl)-N'-((4-(3-methoxycarbonylphenyl)thiazol-2-yl)aminothiocarbonyl)-piperazine,
N-(2,6-bis(pyrrolidin-1-yl)pyrimidin-4-yl)-N'-((4-(pyridin-3-yl)thiazol-2-yl)aminothiocarbonyl)piperazine,
N-(4,6-bis(pyrrolidin-1-yl)-1,3,5-triazin-2-yl)-N'-((4-(pyridin-3-yl)thiazol-2-yl)aminothiocarbonyl)piperazine,
N-methyl-N'-((4-(pyridin-3-yl)thiazol-2-yl)aminothiocarbonyl)piperazine, m.p. of hydrochloride 249-252 ° C,
N-(2,6-bis(pyrrolidin-1-yl)pyrimidin-4-yl)-N'-((4-(3,4-dichlorophenyl)thiazol-2-yl)aminothiocarbonyl)piperazine,
N-(4,6-bis(pyrrolidin-1-yl)-1,3,5-triazin-2-yl)-N'-((4-(3,4-dichlorophenyl)thiazol-2-yl)aminothiocarbonyl)-piperazine,
N-methyl-N'-((4-(3,4-dichlorophenyl)thiazol-2-yl)aminothiocarbonyl)piperazine,
N-(2,6-bis(pyrrolidin-1-yl)pyrimidin-4-yl)-N'-((4-(4-trifluoromethylphenyl)thiazol-2-yl)aminothiocarbonyl)-piperazine,
N-(4,6-bis(pyrrolidin-1-yl)-1,3,5-triazin-2-yl)-N'-((4-(4-trifluoromethylphenyl)thiazol-2-yl)aminothiocarbonyl)-piperazine,
N-methyl-N'-((4-(4-trifluoromethylphenyl)thiazol-2-yl)aminothiocarbonyl)piperazine, m.p. 217-222 ° C,
N-(2,6-bis(pyrrolidin-1-yl)pyrimidin-4-yl)-N'-((4-(3-trifluoromethylphenyl)thiazol-2-yl)aminothiocarbonyl)-piperazine,
N-(4,6-bis(pyrrolidin-1-yl)-1,3,5-triazin-2-yl)-N'-((4-(3-trifluoromethylphenyl)thiazol-2-yl)aminothiocarbonyl)-piperazine,
N-methyl-N'-((4-(3-trifluoromethylphenyl)thiazol-2-yl)aminothiocarbonyl)piperazine.

## EXAMPLE 6

By substituting, in the procedure of Example 1, thiocarbonyl diimidazole to carbonyldiimidazole, the following thioureas are prepared starting from the suitable 2-amino-4-arylthiazoles and from the suitable N-substituted-piperazines:

N-(3,6-bis-diethylamino-pyridin-2-yl)-N'-(4-(4-hydroxyphenyl)thiazol-2-yl)aminothiocarbonyl)piperazine;
N-(3,6-bis-diethylamino-pyridin-2-yl)-N'-(4-(4-hydroxy-3,5-diterbutylphenyl)thiazol-2-yl)aminothiocarbonyl)-piperazine, m.p. of hydrochloride 202-204° C;
N-(3,6-bis-diethylamino-pyridin-2-yl)-N'-(4-(3,4-dihydroxyphenyl)thiazol-2-yl)aminothiocarbonyl)piperazine;
N-(2,6-bis(pyrrolidin-1-yl)pyrimidin-4-yl)-N'-(4-(4-hydroxyphenyl)thiazol-2-yl)aminothiocarbonyl)piperazine;
N-(2,6-bis(pyrrolidin-1-yl)pyrimidin-4-yl)-N'-(4-(4-hydroxy-3,5-ditertbutylphenyl)thiazol-2-yl)-aminothiocarbonyl)piperazine, m.p. 230-233° C;
N-(4,6-bis(pyrrolidin-1-yl)pyrimidin-2-yl)-N'-(4-(4-hydroxy-3,5-ditertbutylphenyl)thiazol-2-yl)-aminothiocarbonyl)piperazine, m.p. 237-240° C;
N-(2,6-bis(pyrrolidin-1-yl)pyrimidin-4-yl)-N'-(4-(3,4-dihydroxyphenyl)thiazol-2-yl)aminothiocarbonyl)-piperazine;
N-(2,6-bis(pyrrolidin-1-yl)pyrimidin-4-yl)-N'-(4-(2,3-dihydroxyphenyl)thiazol-2-yl)aminothiocarbonyl)-piperazine, m.p. 255-260° C;
N-(4,6-bis(pyrrolidin-1-yl)-1,3,5-triazin-2-yl)-N'-(4-(4-hydroxyphenyl)thiazol-2-yl)aminothiocarbonyl)piperazine;
N-(4,6-bis(pyrrolidin-1-yl)-1,3,5-triazin-2-yl)-N'-(4-(4-hydroxy-3,5-ditertbutylphenyl)thiazol-2-yl)-aminothiocarbonyl)piperazine;
N-(4,6-bis(pyrrolidin-1-yl)-1,3,5-triazin-2-yl)-N'-(4-(3,4-dihydroxyphenyl)thiazol-2-yl)aminothiocarbonyl)-piperazine;
N-(4,6-bis(pyrrolidin-1-yl)-1,3,5-triazin-2-yl)-N'-(4-(2,3-dihydroxyphenyl)thiazol-2-yl)aminothiocarbonyl)-piperazine.

## EXAMPLE 7

Following the procedure of Example 1, the following ureas are prepared starting from 2-amino-4-arylthiazoles and from piperidine, morpholine, 4-thiamorpholine.

N-((4-phenylthiazol-2-yl)aminocarbonyl)piperidine, m.p. 144-147° C,
N-((4-phenylthiazol-2-yl)aminocarbonyl)morpholine,
N-((4-phenylthiazol-2-yl)aminocarbonyl)thiamorpholine, m.p. 163-165° C,
N-((4-(4-methoxyphenyl)thiazol-2-yl)aminocarbonyl)piperidine,
N-((4-(4-methoxyphenyl)thiazol-2-yl)aminocarbonyl)morpholine,
N-((4-(4-methoxyphenyl)thiazol-2-yl)aminocarbonyl)thiamorpholine, m.p. 170-172° C,
N-((4-(3-methoxyphenyl)thiazol-2-yl)aminocarbonyl)piperidine,
N-((4-(3-methoxyphenyl)thiazol-2-yl)aminocarbonyl)morpholine,
N-((4-(3-methoxyphenyl)thiazol-2-yl)aminocarbonyl)thiamorpholine,
N-((4-(4-chlorophenyl)thiazol-2-yl)aminocarbonyl)piperidine,
N-((4-(4-chlorophenyl)thiazol-2-yl)aminocarbonyl)morpholine,
N-((4-(4-chlorophenyl)thiazol-2-yl)aminocarbonyl)thiamorpholine, m.p. 160-161° C,
N-((4-(3-chlorophenyl)thiazol-2-yl)aminocarbonyl)piperidine,
N-((4-(3-chlorophenyl)thiazol-2-yl)aminocarbonyl)morpholine,
N-((4-(3-chlorophenyl)thiazol-2-yl)aminocarbonyl)thiamorpholine,
N-((4-(2-chlorophenyl)thiazol-2-yl)aminocarbonyl)piperidine,
N-((4-(2-chlorophenyl)thiazol-2-yl)aminocarbonyl)morpholine,
N-((4-(2-chlorophenyl)thiazol-2-yl)aminocarbonyl)thiamorpholine,
N-((4-(4-fluorophenyl)thiazol-2-yl)aminocarbonyl)piperidine,
N-((4-(4-fluorophenyl)thiazol-2-yl)aminocarbonyl)morpholine,
N-((4-(4-fluorophenyl)thiazol-2-yl)aminocarbonyl)thiamorpholine, m.p. 177-180° C,
N-((4-(3-bromophenyl)thiazol-2-yl)aminocarbonyl)piperidine,
N-((4-(3-bromophenyl)thiazol-2-yl)aminocarbonyl)morpholine,
N-((4-(3-bromophenyl)thiazol-2-yl)aminocarbonyl)thiamorpholine,
N-((4-(4-nitrophenyl)thiazol-2-yl)aminocarbonyl)piperidine,
N-((4-(4-nitrophenyl)thiazol-2-yl)aminocarbonyl)morpholine, m.p. 192-194° C,
N-((4-(4-nitrophenyl)thiazol-2-yl)aminocarbonyl)thiamorpholine, m.p. 197-200° C,
N-((4-(3-nitrophenyl)thiazol-2-yl)aminocarbonyl)piperidine,

N-((4-(3-nitrophenyl)thiazol-2-yl)aminocarbonyl)morpholine,
N-((4-(3-nitrophenyl)thiazol-2-yl)aminocarbonyl)thiamorpholine,
N-((4-(4-(phenylsulphonyl)phenyl)thiazol-2-yl)aminocarbonyl)piperidine,
N-((4-(4-(phenylsulphonyl)phenyl)thiazol-2-yl)aminocarbonyl)morpholine,
N-((4-(4-(phenylsulphonyl)phenyl)thiazol-2-yl)aminocarbonyl)thiamorpholine, m.p. 178-181 °C,
N-((4-(4-(2'-chlorophenylsulphonyl)phenyl)thiazol-2-yl)aminocarbonyl)piperidine,
N-((4-(4-(2'-chlorophenylsulphonyl)phenyl)thiazol-2-yl)aminocarbonyl)morpholine,
N-((4-(4-(2'-chlorophenylsulphonyl)phenyl)thiazol-2-yl)aminocarbonyl)thiamorpholine,
N-((4-(4-aminophenyl)thiazol-2-yl)aminocarbonyl)piperidine,
N-((4-(4-aminophenyl)thiazol-2-yl)aminocarbonyl)morpholine,
N-((4-(4-aminophenyl)thiazol-2-yl)aminocarbonyl)thiamorpholine, m.p. 200-204 °C,
N-((4-(3-aminophenyl)thiazol-2-yl)aminocarbonyl)piperidine,
N-((4-(3-aminophenyl)thiazol-2-yl)aminocarbonyl)morpholine,
N-((4-(3-aminophenyl)thiazol-2-yl)aminocarbonyl)thiamorpholine,
N-((4-(2-aminophenyl)thiazol-2-yl)aminocarbonyl)piperidine,
N-((4-(2-aminophenyl)thiazol-2-yl)aminocarbonyl)morpholine,
N-((4-(2-aminophenyl)thiazol-2-yl)aminocarbonyl)thiamorpholine, m.p. 212-214 °C,
N-((4-(4-((pyrrolidin-1-yl)methyl)phenyl)thiazol-2-yl)aminocarbonyl)piperidine,
N-((4-(4-((pyrrolidin-1-yl)methyl)phenyl)thiazol-2-yl)aminocarbonyl)morpholine,
N-((4-(4-((pyrrolidin-1-yl)methyl)phenyl)thiazol-2-yl)aminocarbonyl)thiamorpholine, m.p. of hydrochloride 220-222 °C,
N-((4-(4-methoxycarbonylphenyl)thiazol-2-yl)aminocarbonyl)piperidine,
N-((4-(4-methoxycarbonylphenyl)thiazol-2-yl)aminocarbonyl)morpholine,
N-((4-(4-methoxycarbonylphenyl)thiazol-2-yl)aminocarbonyl)thiamorpholine,
N-((4-(4-trifluoromethylphenyl)thiazol-2-yl)aminocarbonyl)piperidine,
N-((4-(4-trifluoromethylphenyl)thiazol-2-yl)aminocarbonyl)morpholine,
N-((4-(4-trifluoromethylphenyl)thiazol-2-yl)aminocarbonyl)thiamorpholine,
N-((4-(3,4-dichlorophenyl)thiazol-2-yl)aminocarbonyl)piperidine, m.p. 155-158 °C,
N-((4-(3,4-dichlorophenyl)thiazol-2-yl)aminocarbonyl)morpholine,
N-((4-(3,4-dichlorophenyl)thiazol-2-yl)aminocarbonyl)thiamorpholine, m.p. 162-163 °C.

## Claims

1. Compounds of general formula (I)

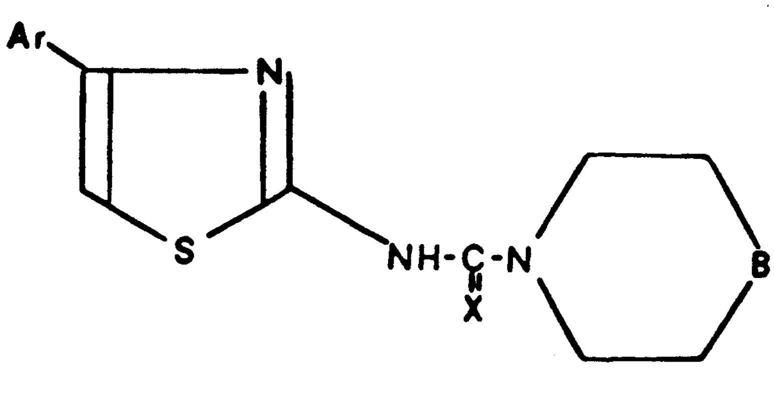

(I)

wherein:
**X** is oxygen or sulfur;
**B** is $CH_2$, oxygen, sulfur or N-R;
**R** is $C_1$-$C_6$ alkyl, phenyl optionally substituted with halogen in o-, m- or p-position; benzyl optionally substituted with halogen in the o-, m- or p- positions; bis(phenyl)methyl; bis(o-,m- or p-halophenyl)-methyl; a 5-6 membered heterocycle having 1 to 3 nitrogen atoms, optionally substituted with 1-2 amino groups, mono-$C_1$-$C_6$-alkylamino, mono-$C_3$-$C_7$-alkenyl- or mono-$C_3$-$C_7$-alkynylamino, di-$C_1$-$C_6$-alkylamino, $C_1$-$C_6$-alkyl-$C_3$-$C_7$-alkenylamino, piperidin-1-yl, morpholin-4-yl, pyrrolidin-1-yl,
and the pharmaceutically acceptable salts thereof.

2. Compounds according to claim 1 in which Ar is selected from phenyl, phenyl substituted with 1 or 2 hydroxy groups, alkoxy and/or $C_1$-$C_6$ alkyl, halogen, amino, phenylthio, phenylsulphonyl, cyano, nitro, alkoxycarbonyl.

3. Compounds according to claim 1 or 2 in which Ar is selected from 4-hydroxyphenyl, 2,3-dihydroxyphenyl, 3,4-dihydroxyphenyl, 4-hydroxy-3,5-di-tert-butylphenyl, 2-, 3- or 4-methoxyphenyl, 2-chlorophenyl, 3-chlorophenyl, 4-chlorophenyl, 2-, 3- or 4-fluorophenyl, 2-, 3- or 4-bromophenyl, 3- or 4-trifluoromethylphenyl, 3-or 4-cyanophenyl, 4-nitrophenyl, 3-nitrophenyl, 3- or 4-phenylsulphonyl-phenyl, 3- or 4-(2'-chlorophenyl)sulphonylphenyl, 4-phenylthiophenyl, 4-((pyrrolidin-1-yl)methyl)phenyl, 2-, 3- or 4-aminophenyl, 3-or 4-methoxycarbonylphenyl, $\beta$-pyridyl, 3,4-dichlorophenyl, 2,3-dihydroxyphenyl and R is selected from pyridin-2-yl, pyrimidin-4-yl, pyrimidin-2-yl or 1,3,5-triazin-2-yl, which can optionally be substituted with 1 or 2 amino, mono-$C_1$-$C_6$-alkylamino, 2-propenylamino, 2-propynylamino, propylamino, isopropylamino, dimethylamino, diethylamino, ethyl-2-propenylamino, pyrrolidino groups.

4. Compounds according to claims 1-3, in which R is selected from [2,6-bis(diethylamino)pyrimidin-4-yl], [2,6-bis(2-propenylamino)pyrimidin-4-yl], [2,6-bis(amino)pyrimidin-4-yl], [2,6-bis(pyrrolidin-1-yl)-pyrimidin-4-yl], [4,6-bis(pyrrolidin-1-yl)-pyrimidin-2-yl], [4,6-bis(2-propenylamino)-1,3,5-triazin-2-yl], [4,6-bis(diethylamino)-1,3,5-triazin-2-yl], [4,6-bis(pyrrolidin-1-yl)-1,3,5-triazin-2-yl], [3,6-bis(diethylamino)-pyridin-2-yl], [3,6-bis(pyrrolidin-1-yl)pyridin-2-yl], [3,6-bis(2-propenylamino)pyridin-2-yl], methyl, phenyl, p-fluorophenyl, p-bromophenyl, p-chlorophenyl, bis(phenyl)methyl, bis(p-fluorophenyl)methyl, bis(p-chlorophenyl), bis(p-bromophenyl).

5. A process for the preparation of the compounds according to claims 1-4, which process comprises reacting a compound of formula (II):

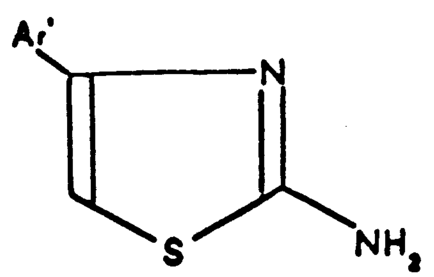

(II)

wherein Ar' has the same meanings as Ar in claims 1-3 or it is a group which can be converted into Ar by removing any protective groups present, first with carbonyldiimidazole or simylar difunctional carbonylating reagents, then with piperazines of formula (III):

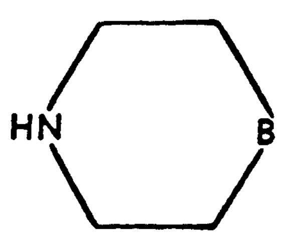

(III)

wherein B is as defined above.

6. Pharmaceutical compositions containing one compound of claims 1-4 as the active ingredient, in admixture with pharmaceutically acceptable carriers or excipients.

7. The use of the compounds of claims 1-4 as therapeutical agents.

8. The use of the compounds of claims 1-4 for the preparation of a medicament for the treatment of

bronchial hyper-reactivity.

European Patent Office

# EUROPEAN SEARCH REPORT

Application Number

EP 92 11 0267

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 006 368 (PIERRE FABRE S.A.)<br>* page 5, line 10 - page 7, line 14; claims 1,4,5 *<br>--- | 1,6-8 | C07D417/12<br>A61K31/425<br>C07D277/48<br>A61K31/435<br>A61K31/505<br>A61K31/53 |
| A | EP-A-0 005 070 (PFIZER INC.)<br>* claims 1,8,9 *<br>--- | 1,6-8 | |
| D,A | EP-A-0 069 154 (MITSUI TOATSU K.K.K.)<br>* claims 1,5-8 *<br>--- | 1,6-8 | |
| A | FR-A-1 068 631 (AMERICAN CYANAMID)<br>* the whole document *<br>--- | 1,6-8 | |
| D,A | EP-A-0 069 784 (MITSUI TOATSU K.K.K.)<br>----- | 1,6-8 | |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 15 SEPTEMBER 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document